# EUROPEAN PATENT SPECIFICATION

(11) **EP 2 197 436 B1**
(45) Date of publication and mention of the grant of the patent: **22.11.2017**
(21) Application number: 08805430.9
(22) Date of filing: 12.09.2008
(51) Int. Cl.: A61K 31/198, A61K 38/05, A24D 3/14, A61P 25/32, A61P 25/34

(54) **SUCKING TABLET FOR USE IN REDUCING TOBACCO AND/OR ALCOHOL DEPENDENCE**
LUTSCHTABLETTE ZUR VERWENDUNG IN DER VERRINGERUNG DER TABAK- UND/ODER ALKOHOLABHÄNGIGKEIT
PASTILLE POUR UNE UTILISATION DANS LA RÉDUCTION DE LA DÉPENDANCE AU TABAC ET/OU À L'ALCOOL

(30) Priority: 14.09.2007 FI 20070705
(43) Date of publication of application: 23.06.2010
(73) Proprietor: BIOHIT OYJ, 00880 Helsinki (FI)
(72) Inventor: SUOVANIEMI, Osmo, 00570 Helsinki (FI); SALASPURO, Mikko, 00260 Helsinki (FI); SALASPURO, Ville, 00029 Hus (FI); MARVOLA, Martti, 00660 Helsinki (FI)
(74) Representative: Seppo Laine Oy
(86) International application number: PCT/FI2008/050507
(87) International publication number: WO 2009/034232

(56) References cited:
- EP-A1- 1 304 048
- EP-A2- 1 256 283
- WO-A1-02/36098
- WO-A1-2006/037848
- CN-A- 1 311 022
- CN-A- 1 593 395
- RU-C2- 2 201 247
- US-A- 4 532 947
- TALHOUT REINSKJE ET AL: "Role of acetaldehyde in tobacco smoke addiction" EUROPEAN NEUROPSYCHOPHARMACOLOGY vol. 17, no. 10, 23 March 2007 (2007-03-23), pages 627-636, XP002596455 ISSN: 0924-977X Retrieved from the Internet: URL:http://dx.doi.org/10.1016/j.euroneuro. 2007.02.013 [retrieved on 2010-08-13]
- MCBRIDE WILLIAM J ET AL: "Involvement of acetaldehyde in alcohol addiction" ALCOHOLISM CLINICAL AND EXPERIMENTAL RESEARCH, vol. 26, no. 1, January 2002 (2002-01), pages 114-119, XP002596456 ISSN: 0145-6008
- CALLAWAY JAMES C ET AL: "Formation of tetrahydroharman (1-methyl-1,2,3,4-tetrahydro-beta-carbo line) by Helicobacter pylori in the presence of ethanol and tryptamine" LIFE SCIENCES, vol. 58, no. 21, 1996, pages 1817-1821, XP002596457 ISSN: 0024-3205
- MARTIN G.J. ET L.: 'The effect of cysteine in modifying the action of ethanol given chronically in rats' LIFE SCIENCES vol. 5, no. 24, 1966, pages 2357 - 2362, XP023765611
- BAKER D.A. ET AL.: 'Neuroadaptations in cystine-glutamate exchange underlie cocaine relapse' NATURE NEUROSCIENCE vol. 6, 2003, pages 743 - 749, XP008132286
- SPRINCE H. ET AL.: 'Protective action of ascorbic acid and sulphur compunds against aldehyde toxicity:implications in alcoholism and smoking' AGENTS AND ACTIONS vol. 5, no. 2, 1975, pages 164 - 173, XP009137510
- KALIVAS P.W. ET AL.: 'Glutamate transmission in addiction' NEUROPHARMACOLOGY vol. 56, no. 1, 2008, pages 169 - 173, XP008132287

## Description

Smoking causes various effects that are detrimental to health. It increases, to an extremely significant extent, the risk of various cancers, such as cancer of the lungs, the mouth, the pharynx, the larynx, the oesophagus, and the stomach. Smoking causes coronary heart disease and chronic obstructive pulmonary disease. Smoking during pregnancy causes an increase in the rate of foetal and neonatal deaths, premature labour, and the low birth weights of new-borns. Children, whose parents smoke, suffer more often from bronchitis and pneumonia and have an increased risk of developing cancer later in their lives.

In spite of being well aware of the various severe health hazards, smokers find it extremely difficult to stop smoking. There are about 1.3 billion smokers in the world. Even if they tried to stop smoking by means of the nicotine replacement therapy, only about 20% would be successful in withdrawing from tobacco over a period of 12 months (Rose, 2006, Nicotine and nonnicotine factors in cigarette addiction. Nicotine Tob. Res. 3, 383-390 and Wil-lemsen et al., 2003, Ned. Tijdschr. Geneeskd.) It has been estimated that smoking causes 5 million deaths a year in the world, mainly due to lung cancer, chronic obstructive pulmonary disease and cardiovascular diseases (Ezzati and Lopez, 2003, The Lancet 362: 847-852).

Alcohol is also known to cause cancer. According to Salaspuro (Salaspuro, M. Best Pract Res Clin. Gastroenterol (2003) 17:679-94) and Francheschi et al. (Cancer Res (1990) 50:6502-07), smoking and alcohol together, multiply the risks of developing cancers of the upper alimentary tract by 150-fold. It has been assessed that about 2 million people a year develop cancers of the upper alimentary tract, which are mainly caused by smoking and drinking alcohol.

Finding a method, composition or programme, which would enable the smokers to stop smoking and heavy users of alcohol to refrain from using alcohol, would be of an enormous consequence to the national health.

Acetaldehyde has been observed to cause cancer in animals and to also comprise a local carcinogen, when occurring in human saliva and the alimentary tract.

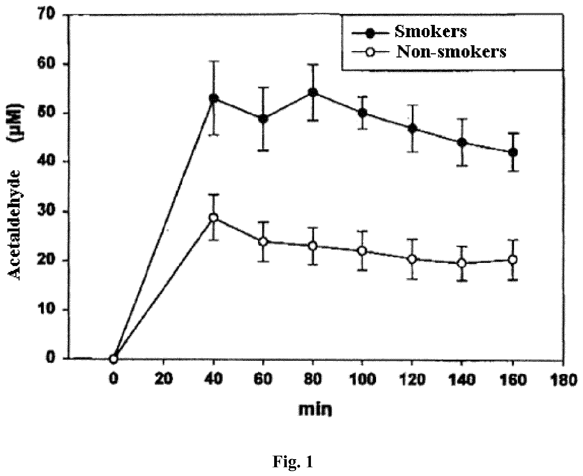

Salaspuro et al. 2004 (Salaspuro V, Salaspuro M. Synergistic effect of alcohol drinking and smoking on in vivo acetaldehyde concentration in saliva. Int J Cancer. 2004 September 10; 111 (4): 480-3) have proven that the average content of acetaldehyde in saliva in vivo of smokers, even without smoking, after enjoying ethanol, is about two times higher than in non-smokers throughout an entire control period of 160 minutes (Fig. 1). The area under the graph that describes the acetaldehyde in the smokers' saliva was considerably larger than for the non-smokers, 114.8 ± 11,5µM x h compared with the value 54.2 ± 8.7µM x h, (p = 0.002), respectively.

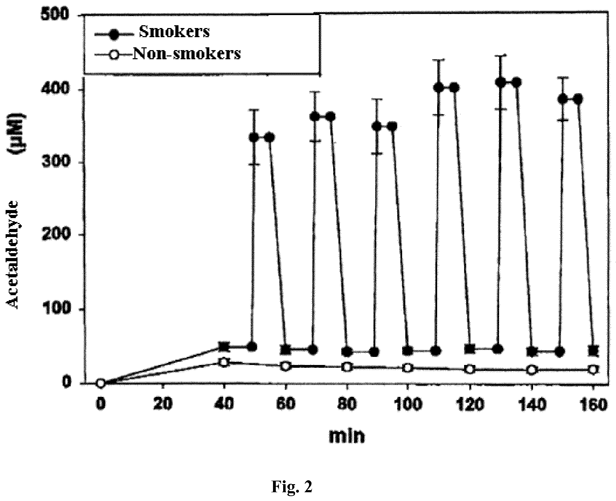

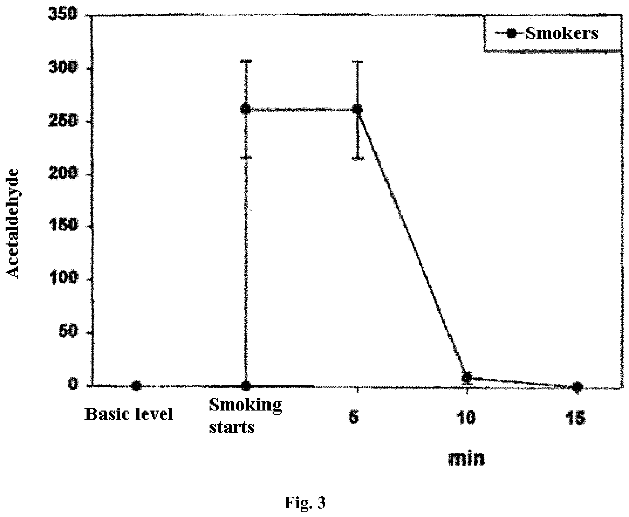

During a period of smoking a cigarette, the acetaldehyde in saliva in vivo increased by tenfold from the levels that occurred when enjoying ethanol alone. The acetaldehyde in saliva immediately increased, when smoking began, but also quickly decreased after smoking stopped (Fig. 2). The area below the graph that describes the acetaldehyde in smokers was seven times larger when compared to the non-smokers, the difference being significant, 369.5 ± 12.2µM x h, respectively, compared with the value 54.2 ± 8.7µM x h, (p = 0.001). During active smoking, the acetaldehyde in saliva increased from its basic level to a value of 261.4 ± 45.5µM. The acetaldehyde in saliva immediately increased, when smoking began, but also quickly decreased after smoking stopped (Fig. 3).

The first metabolism product of alcohol is acetaldehyde. Alcohol is evenly distributed in the liquid phase of the organism. Consequently, as long as an individual is enjoying alcohol and as long as there is alcohol in the organism, the amount of alcohol in the blood, saliva, gastric juice, and the contents of the bowel remain the same. The microbes of the alimentary tract are capable of oxidizing alcohol into acetaldehyde in the alimentary tract.

Acetaldehyde is formed in the organism from alcohol as a result of the hepatic metabolism and, locally, in the alimentary tract through microbes (Salaspuro et al, (1996) Ann Med 28:195-200). On the other hand, carcinogenic acetaldehyde can be formed endogenously by the microbes in the mouth from various foodstuffs, which have high sugar or hydrocarbon contents, particularly in an acid-free stomach. Atrophic gastritis and an acid-free stomach (achlorhydria) are well-known risk factors for the cancer of the stomach.

Due to the microbial metabolism, acetaldehyde is formed in the stomach in a case, where the stomach is acid-free or rendered acid-free by means of medicines (Väkeväinen et al., 2000, Alimentary Pharmacology Ther 14:1511-1518). In patients suffering from atrophic gastritis, microbes produce high acetaldehyde contents from ethanol and sugar in the stomach, resulting in an increased risk of developing cancers of the stomach (Väkeväinen et al, Scand J Gastroenterol 2002 (6):648-655).

No alcohol fermentation takes place in an acidic stomach. On the other hand, a *Helicobacter pylori* infection and certain medicines, such as the protein pump inhibitor (PPI) increase the pH of the stomach.

The patent application WO 02/36098 describes a preparation that slowly dissolves in the mouth, the stomach or the large intestine, releasing, over a long period of time, a substance that binds acetaldehyde and, in this way, reducing the risk of developing cancers of the mouth and the alimentary tract.

The patent application WO 2006/037848 describes preparations that are kept in the mouth or attached to a cigarette, or compositions, which are absorbed into or in some other way attached to the cigarette, the surface that is kept in the mouth, in particular, and which are used for the time it takes to consume the tobacco product. In that case, the acetaldehyde-binding substance is released into the saliva from the preparation that is kept in the mouth or from the tobacco product during the use of the tobacco product.

By means of the compositions and the preparations of the patent specifications mentioned above, it is possible to reduce the exposure of individuals to carcinogenic acetaldehyde.

Martin et al., Life Sciences 1966, vol.5(24), pages 2357-2362, discuss the effect of L-cysteine in modifying the action of ethanol given chronically to rats, wherein the L-cysteine is given orally to the rats as part of their diet.

Surprisingly, in connection with the present invention, it has been observed that the same or corresponding preparations, which release the acetaldehyde-binding compound into the mouth or the alimentary tract, can also be used for withdrawing from the use of tobacco and alcohol. In tests related to the present invention, it was observed that the testees no longer had a craving for tobacco, a pipe or cigars, when the acetaldehyde coming into the mouth during smoking and/or enjoying alcohol was bound. With the lack of the feeling of enjoyment, the testees gradually give up consuming cigarettes and alcohol.

The present invention provides a preparation, which can be used for withdrawing from tobacco and/or alcohol. The preparation removes an essential portion of the acetaldehyde that is present in the smoke of the tobacco product, or binds the acetaldehyde, which enters the saliva or the alimentary tract in connection with smoking or drinking alcohol.

It is also disclosed herein a method, by means of which a person can withdraw from tobacco and/or alcohol.

The method also preferably comprises a programme, which makes the withdrawal from tobacco and/or alcohol successful.

To be more precise, the sucking tablet according to the invention is characterized by what is presented in the characterizing part of Claim 1.
Fig. 1 shows the salivary acetaldehyde in vivo after ethanol ingestion in smokers (without simultaneous smoking) and in non-smokers.
Fig. 2 shows the salivary acetaldehyde in vivo after ethanol ingestion in smokers (with simultaneous smoking) and in non-smokers. Differences between the acetaldehyde concentrations are significant at all time points from 40 to 160 min (p≤0.05).
Fig. 3 indicates the salivary acetaldehyde in smokers after smoking one cigarette (without simultaneous alcohol drinking).
Fig. 4 shows the salivary acetaldehyde (SEM) after 5 min of smoking with a placebo, and with sucking tablets containing 1.25 mg, 2.5 mg, 5 mg or 10 mg of L-cysteine.
Fig. 5 shows the capability of acetyl cysteine to bind acetaldehyde compared with a placebo on testees, who had consumed alcohol.

### Detailed description of the invention

The present invention provides sucking tablets in accordance with the appended claims for use in reducing tobacco and/or alcohol dependence, wherein the use comprises stages a), b) and c) as defined in appended claim 1 Stage a) should be repeated in connection with smoking or consuming alcohol for at least 5, preferably at least 10, more preferably 15 to 50 times, or as many as 20 to 100 times, typically 10 to 20 times.

One stage of the treatment or the programme comprises observing that smoking and/or alcohol dependence has reduced or ended. This can be carried out directly after undergoing the programme. If no withdrawal has taken place, the individual is advised to repeat the programme. In case withdrawal seems to have taken place, i.e., the individual no longer has a craving for tobacco and/or alcohol, checkups are carried out at regular intervals, first at intervals of 2 to 7 days, then, e.g., 2 weeks, and then one month. The withdrawal can be considered successful, if the individual has been able to refrain from regular smoking and/or regular and heavy alcohol consumption (exceeding moderate use) for the next half a year or preferably a year.

Furthermore, it is preferable to repeat the smoking and/or alcohol drinking activity by using the withdrawal preparation in a way otherwise similar to the way the individual normally smokes or consumes alcohol. Extinguishing the craving for alcohol in normal drinking occasions by means of opiate antagonists is described in patent US 4,882,335.

The use described herein also comprises a stage, where the individual is told about the health hazards and dependence caused by the acetaldehyde contained in tobacco and/or alcohol so that, when starting the method, they are aware of the damages caused by tobacco and/or alcohol.

The reduction in tobacco and alcohol dependence described herein can be a consequence of the fact that, when binding the acetaldehyde into a form harmless to the organism, no products that cause dependence can be formed therefrom. According to Talhout et al., European Neuropsychopharmacology 2007, vol.17, pages 627-636, products, such as harmane and salsosinol, which are generated as condensation products from acetaldehyde and biogenous amines, may be substances that cause acetaldehyde dependence. Harmane is formed when smoking and the harmane levels in the blood of smokers are 2 to 10 times higher than those of non-smokers. Easily exceeding the blood-brain barrier and having enough potential to inhibit monoamine oxidase, harmane can affect the lower monoamine oxidase (MAO) activity observed in smokers' brains. Harmane and salsosinol inhibit monoamine oxidase (MAO). Talhout et al. have concluded that acetaldehyde may increase the potential of tobacco products to cause addiction, when acetaldehyde and biogenous amines are formed in cigarette smoke and/or in vivo. Talhout et al. 2007 have discovered that acetaldehyde causes dependence in rodents. This dependence cooperates with the dependence caused by nicotine.

McBride et al. (2002), in turn, have suggested that salsosinol enhance the craving for alcohol and, through that, the alcohol dependence. Naoi et al., NeuroToxicology 25 (2003) 193-204 and Haber et al. 1996, Alcohol Clin. Exp. Res. 20(1):87-92 have also discussed the effects of salsosinol.

Acetaldehyde may penetrate the blood-brain barrier and, on the other hand, it can also be formed in the brain. However, studies are controversial, as normal people do not have measurable amounts of acetaldehyde in their blood while burning alcohol. The brain studies, in turn, have been run on test animals and very high acetaldehyde concentrations were used.

The acetaldehyde, which is dissolved in saliva from tobacco or formed in saliva from alcohol as a combustion product of microbes, can condense with amino acid tryptamine and form harmane that functions as a neurotransmitter. Harmane, the condensation product of acetaldehyde and tryptamine, can also be formed in an acid-free stomach in connection with using drugs that inhibit the acid secretion and as a result of the action of the ADH enzyme contained in Helicobacter, whenever the individual either consumes alcohol or when alcohol drifts into the saliva or gastric juice through the blood circulation. Tryptamine occurs in milk products (e.g., fermented cheese) and soy products, for example. The harmane that is generated in the mouth or the stomach can absorb through the mucous membranes directly into the blood circulation and further drift to the brain without being subjected to the detoxication mechanisms of the liver in between. The harmane that enters the brain can cause dysphoric symptoms (resembling a hangover), or it may also enhance the alcohol dependence (Callaway et al. 1996, Life Sciences 58(21):1817-1821).

Binding acetaldehyde from the saliva or preventing it from entering the saliva considerably reduces the formation of harmane and any other wrong neurotransmitters and their entering the brain. The preparations according to the invention, and the use thereof, also contribute to reducing the symptoms of hangover.

The use of the products according to the invention for withdrawing from tobacco and/or alcohol can be combined with the previously known methods that have been used for withdrawing from tobacco. Tobacco withdrawal products that typically contain nicotine, such as nicotine gum, can be used at the times, when the individual is able to refrain from smoking. Such products include, e.g., Nicorette ® products, such as chewing gums, tablets, plasters or products, which are described, for example, in the patent specifications US 5,488,962 and US 5,845,647. If the individual is unable to refrain from the use of tobacco products and restarts smoking, they can use preparations that contain the acetaldehyde-binding compounds according to the invention, which contain the compounds capable of binding acetaldehyde.

The use presented herein for withdrawing from tobacco and/or alcohol thus includes a stage(s), wherein the individual is withdrawn from the dependence caused by acetaldehyde by means according to the invention; and a stage(s), wherein the individual is withdrawn from nicotine by means of tobacco withdrawal products that contain nicotine. The products according to the invention are used during the time, when the individual is not able to refrain from smoking, and the nicotine-containing preparations during the time, when the individual is able to refrain from smoking.

The "tobacco product" refers to any tobacco product, such as a cigarette, cigar or pipe. The tobacco product can already include a filter that is normally used or the product can be without a filter. However, it is preferable that the filter does not prevent the smoke from drifting through the component (holder, acetaldehyde-binding filter) that contains the acetaldehyde-binding material.

"Smoking" refers to the use of the tobacco product, such as the cigarette, cigar or other tobacco product.

"In connection with smoking" herein refers to the period of time that starts from starting to smoke and ends, when smoking stops. In particular, "in connection with smoking" herein refers to the period of time, when acetaldehyde enters the mouth of the smoker.

"In connection with consuming alcoholic drinks" herein refers to the period of time that starts from starting to consume alcohol and ends, when there is no more alcohol in the blood.

### Binding of acetaldehyde

"Binding of acetaldehyde" refers to a chemical reaction between the acetaldehyde and a compound that has a free amino group and/or sulphhydryl or sulphonic group, in which reaction the acetaldehyde together with the "acetaldehyde-binding substance" forms a larger molecule and in which reaction water can be formed. The "acetaldehyde-binding substance" preferably refers to a compound that comprises one or more free amino groups and sulphhydryl groups or sulphonic groups. The "compound" can refer to one or more compounds.

For example, when reacting with cysteine, acetaldehyde binds itself to both the sulphhydryl and the amino group, forming 2-methyl-L-thiazolidine-4-carboxylic acid and water. Acetaldehyde can bind itself to the amino group of almost any protein, whereby Schiff base or a 2-methyl-imidzole ring is formed.

Cysteine, its salts and derivatives are particularly suitable for the use described herein. The most suitable amino acids in the use described herein include L- and D-cysteine, acetyl cysteine, N-penicillamine or the derivatives of cysteine, which function in the same way as L- or D-cysteine and their salts. The compound is most suitably L-cysteine and its salts.

Suitable compounds for binding acetaldehyde in the organism also include the compounds according to the formula (I): 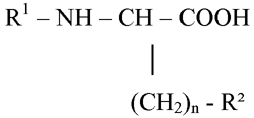 wherein
R¹ is hydrogen or an acyl group that has 1 to 4 carbon atoms;
R² is a sulphhydryl or sulphonic group;
n is 1, 2, 3 or 4.

The amino acids or other compounds that suitably bind acetaldehyde and contain a free sulphhydryl (SH) and/or amino (NH₂) group include:
L-cysteine
D-cysteine
cysteinic acid,
cystine,
cysteine-glycine,
threo- or erythro-β-phenyl-DL-cysteine,
β-tetramethylene-DL-cysteine,
methionine,
serine,
D-penicillamine and its N-terminal dipeptides,
semicarbazide,
glutathione,
reduced glutathione,
β-mercaptoethyl amine
D,L-homocysteine,
DL-homocysteinic acid
N-acetylcysteine,
L-cysteinyl-L-valine,
β-β-tetramethylene-DL-cysteine,
cysteinylglycine,
mercaptoethyl glycine,
cysteine hydrochloride,
thiamine hydrochloride,
sodium metabisulphite,
arginine,
glycine,
lycine,
ammonium chloride,
1,4 ditiothreitol,
mercaptane,
or a salt of any of these compounds.

The acetaldehyde-binding substance according to the present invention is L-cysteine.

The effects of some acetaldehyde-binding or other aldehyde-binding substances can be improved by vitamins, such as L-ascorbic acid.

Suitable compounds also include the salts of the acetaldehyde-binding compounds (pharmaceutically acceptable salts, in particular). Any acetaldehyde-binding compounds that present no health hazards in the dosages used are suitable for the preparations described herein. The acetaldehyde-binding compounds disclosed wherein can also be used to bind aldehydes other than acetaldehyde.

It is also advantageous, if the taste or the smell of the compounds is not unpleasant or too strong. It is possible to disguise the unpleasant taste of an effective compound by means of suitable sweetening agents or flavourings; however, by using compounds that have a mild and/or pleasant taste, the compound can be kept simple and it is easier to produce. Another way of reducing the significance of the product's taste is to use as small amounts as possible.

Tobacco can be used by smoking, chewing, or wetting or snuffing. According to our research, smoking, in particular, seems to cause the formation of acetaldehyde in the mouth. Smoking in connection with the present invention typically refers to smoking cigarettes or cigars or, alternatively, a pipe.

A "harmful/carcinogenic concentration of acetaldehyde" in the human mouth, oesophagus, stomach and large intestine is 50 to 100 µMol/l of saliva. Even lower acetaldehyde concentrations together with tryptamine cause the formation of harmane. A detrimental, carcinogenic or harmane-forming acetaldehyde concentration in the human mouth can be obtained, for example, in connection with smoking and/or drinking alcohol, and even after drinking alcohol as long as there is alcohol in the blood, saliva or gastric juice.

An object of the present invention is to keep the acetaldehyde in the mouth on a level essentially lower than without using the preparations according to the present invention. The acetaldehyde concentration of saliva is preferably kept on a level that is at least 60%, and more preferably at least 80% lower than without using the composition. It is most preferable to remove essentially all of the acetaldehyde so that there is no time to form the harmane that causes addiction. In connection with smoking, all of the acetaldehyde in saliva can be removed by the preparations mentioned above. In connection with using alcohol, the acetaldehyde that is formed in the saliva can preferably be reduced by at least about 70%. Preparations that slowly release cysteine into the stomach can be used to preferably reduce the acetaldehyde by at least about 70%, preferably by at least about 80%, and more preferably by at least about 90%.

The "local preparation that is placed in the mouth" refers to all preparations that are sucked, and wherein the release of the substance is intended to have a local effect in the mouth. The preparation can release the acetaldehyde-binding substance in a short time, e.g., during 5 to 15 minutes, or for a long time, such as more than a half an hour.

The term "composition" herein refers to a composition that comprises the effective substance(s), possibly mixed together with a suitable carrier. The composition is in the form of a local preparation, which is suitable to be used in the mouth.

The "composition" refers to a non-toxic composition suitable for human consumption. The effective compounds also refer to the salts of these compounds; particularly salts that are suitable for human consumption or pharmaceutically acceptable. The compositions according to the invention are particularly suitable for oral use.

In addition to what is called an effective substance(s) that binds acetaldehyde, the preparation preferably comprises at least one carrier, which does not prevent but facilitates the release of the effective substance. In the case of a preparation that slowly releases the effective substance, it is preferable that the carrier adjusts the release of the effective substance. Furthermore, it is preferable that the preparation has a shape that makes it easier to keep in the mouth either during smoking or when drinking alcohol. The preparation can otherwise be of any shape, such as round or elliptical, longitudinal, capsule-shaped, convex or annular. It is also preferable that the preparation is relatively small so that its use does not complicate or change the smoking activity or the use of alcohol.

The ideal operating time of the preparation that is used in connection with smoking is the same as that consumed by smoking (about 5min). The operating time of the preparation, which is used in connection with consuming alcohol and which slowly releases the acetaldehyde-binding compound either into the mouth or the stomach, is preferably 2 to 4 hours.

It is advantageous, if the amount of effective substance can be kept as small as possible, as the taste of the compound does not then need to be disguised at all or needs to be disguised to a minor degree only, if the taste of the substance is unpleasant. The individual using the composition does not need to consume exceedingly high concentrations of the compound. The preparation is also less expensive. The preparation according to the invention comprises 1,25 to 5 mg of L-cysteine.

In addition to L-cysteine, the composition can comprise:
1. Pharmaceutically acceptable diluents (fillers, extenders),
2. Sweeteners, such as sugars and sugar alcohols,
3. Flavourings, and
4. Slip additives/lubricants.

The sugars can comprise, for example, saccharose, fructose or glucose or mixtures thereof. The sugar alcohols can comprise mannitol, sorbitol, maltitol, lactitol, isomalt, or xylitol or mixtures thereof. No additive preferably reacts with the other ingredients in the preparation. Not being too sweet, a preferable sweetener comprises mannitol, and its amount in the preparation can be quite large; accordingly, it functions as a diluent at the same time.

The flavourings can comprise, for example, spearmint, peppermint, menthol, citrus fruit, eucalyptus or aniseed or a mixture thereof.

The preparation can also comprise other ingredients, such as substances that prevent bad oral smell, substances that function as breath fresheners and/or prevent dental caries, or the preparation can comprise vitamins. The preparation can also comprise substances that increase salivation. However, these additives should not prevent the quick release of the acetaldehyde-binding substance into the saliva. As previously described herein, the preparation should release the acetaldehyde-biding substance so effectively that an essential amount of acetaldehyde is bound to the saliva before the acetaldehyde influences the cells of the mucous membrane in the mouth. Also described herein is a preparation (such as one tablet) which can essentially comprise or consist of the following:

| | |
|---|---|
| Acetaldehyde-binding substance(s) | 1 to 50 mg |
| Diluting agent(s)/sweetener(s) | 50 to 750 mg |
| Flavouring(s) | q.s. |
| Lubricant(s) (0.5 to 3% by weight) | 5 to 25 mg |
| | |
| The preparation can be a sucking tablet comprising: | |
| Acetaldehyde-binding substances | 1 to 50 mg |
| Sugar or sugar alcohol, such as mannitol | 50 to 750 mg |
| Flavouring | q.s. |
| Magnesium stearate | 5 to 25 mg |

The composition is prepared by mixing a powdery mass and compressing it into sucking tablets by any well-known methods.

If the amount of acetaldehyde-binding compounds is increased, the amount of diluent(s)/sweetener(s) and flavourings can also be increased, as the taste of the acetaldehyde-binding substances must be disguised.

According to another method described herein, the preparation can essentially comprise or consist of the following:

| | |
|---|---|
| Acetaldehyde-binding substances | 1 to 50 mg |
| Gum base comprising sweeteners or other substances | 500 to 1500 mg |
| Flavouring | q.s. |
| Lubricant (0.5 to 3% by weight) | 5 to 30 mg |

The gum base, which can comprise medicated chewing gum (Morjaria, Y. et al., Drug Delivery Systems & Sciences, vol. 4, No. 1, 2004) that comprises natural or synthetic elastomers, softeners, waxes and lipids. Natural gum bases, including crude rubber and smoked natural rubber, are permitted by the FDA. However, modem gum bases are mostly synthetic and include styrene-butadiene rubber, polyethylene and polyvinyl acetate. The gum base constitutes 15 to 40% of the chewing gum. The remaining portion consists of medicine, sugar, sweeteners, softeners, flavourings and colouring agents.

The majority of the chewing gum-based drug delivery systems are prepared using conventional methods. However, directly compressible powder gums are modem alternatives to the medicated chewing gums. Pharmagum is a compressible new gum system.

It is a mixture of polyol(s) and/or sugars with a gum base. A formulation that contains Pharmagum gums can be compressed into a gum tablet by using conventional tablet presses. The manufacturing method is quick and inexpensive. The amount of gum base in the preparation, comprising sweeteners can be 50 to 500 mg, preferably 500 to 1500 mg.

Pharmagum S contains rubber base and sorbitol, Pharmagum M contains rubber base, mannitol, and isomalt.

The diluents include, e.g., lactose, calcium phosphates, starch, carboxymethyl cellulose, hydroxymethyl cellulose. The sweetener can be, for example, mannitol or xylitol.

The preparation is preferably capable of binding acetaldehyde from the saliva during the smoking of at least one, possibly 1, 2 or 3 cigarettes or cigars.

### Administering the acetaldehyde-binding substance

The content of acetaldehyde formed in the saliva as a consequence of smoking can be reduced so that, in connection with smoking, the preparation, preferably one or two preparations at the same time, are placed in the mouth, under the tongue or in the cheek, or between the cheek and the gum, for example, releasing, at a suitable and preferably constant velocity, cysteine or another acetaldehyde-binding substance that essentially has the same effect as cysteine, continuously and advantageously, until one tobacco product has been consumed. When starting the next tobacco product, a new acetaldehyde-binding preparation is placed in the mouth. According to a preferred embodiment of the invention, the preparation is also capable of reducing the salivary acetaldehyde content during smoking one cigar, cigarette or pipe, to the level where the acetaldehyde was before smoking.

The use of the acetaldehyde-binding substance is repeated as many times as a new tobacco product is started. It is preferable to place the preparation in the mouth already before starting to smoke a new cigar, cigarette or pipe.

The preparation according to a preferred embodiment of the present invention is capable of releasing the acetaldehyde-binding substance into the saliva under the conditions prevailing in the mouth within less than 30 minutes, and preferably within less than 15 minutes from the point of time, when the preparation is brought into contact with the saliva. Accordingly, the acetaldehyde-binding substances are released within 0 to 5 minutes, more preferably within 0 to 10 minutes, most preferably within 0 to 15 minutes from the point of time, when the preparation is brought into contact with the saliva. The release of the acetaldehyde-binding substances preferably takes essentially the time of smoking one cigar or cigarette, i.e., the time of actual smoking and a few minutes longer.

### Preparation that acts in the mouth for a long time

During smoking or drinking alcohol, compositions can be used, which slowly release the acetaldehyde-releasing substance into the mouth and which are described in the patent application WO02/36098. "Prolonged release of the effective substance" means that the substance is released over at least 30 minutes, preferably at least 120 minutes, most preferably more than four hours. Using the compositions according to the invention, release times of the effective substance of as long as 4 to 8 hours can be achieved. The compound is preferably released under the conditions of the mouth in amounts of 15 to 25 mg an hour. 1 or 2 preparations according to the invention can be placed in the mouth at a time and they can be replaced with new ones at 4 to 10-hour intervals, most preferably at 6 to 8-hour intervals.

### Examples

### Example 1 (not according to the invention)

A sucking tablet was prepared, comprising:

| | |
|---|---|
| Cysteine | 20 mg |
| Mannitol (or an equivalent sugar or sugar alcohol) | 750 mg |
| Flavouring | q.s. |
| Magnesium stearate | 10 mg |

The composition was prepared by mixing a powdery mass and compressing it into sucking tablets.

### Example 2

Sucking tablets were prepared as in Example 1, but they comprised 1.25 mg, 2.5 mg, 5 mg, and 10 mg of cysteine.

### Example 3

Two individuals tested the preparation prepared according to Example 1. The salivary acetaldehyde contents of the testees were measured before smoking and then after 5 minutes during smoking, i.e., 0min, 5min, 10min, and 15min after the testees started smoking. Each testee smoked one cigarette and, at the same time, saliva was collected from their mouths, as they sucked placebo tablets. Smoking lasted for 5min. In another test, the testees repeated the study by sucking tablets containing 20 mg of cysteine.

Before smoking, the salivary acetaldehyde content of each testee was very low. In the second test, the acetaldehyde content had reduced to a non-measurable level already after the first 5 minutes.

### Example 4

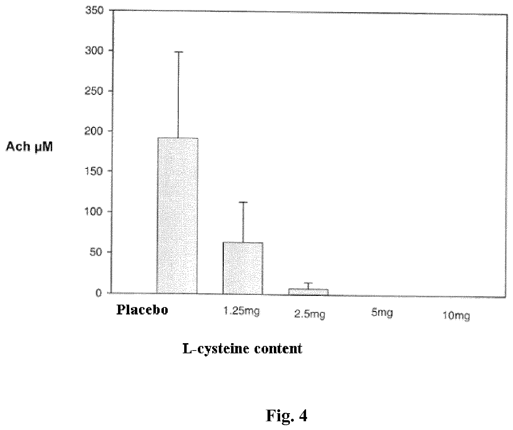

Five smokers (of the age of 29 ± 2.8) participated in the study, in which three cigarettes were smoked (with cleaning periods in between). While smoking each cigarette (in 5 minutes time), the voluntaries sucked tablets blindfold, containing a placebo, 1.25 mg, 2.5 mg, 5 mg, 10 mg or 20 mg of L-cysteine. The acetaldehyde in the saliva samples was analysed by gas chromatography after 0, 5, 10, 20 minutes from starting to smoke. The L-cysteine tablets (5 mg, 10 and 20 mg) removed from the saliva all of the acetaldehyde originating from tobacco (see Fig. 4). The average salivary acetaldehyde contents immediately after smoking were 191.2 ± 48.5 µM, 0 µM, 0 µM, 0 µM with the placebo and the 5 mg, 10 mg, and 20 mg L-cysteine tablets, respectively.

The study showed that even 5 mg of L-cysteine, when delivered with a melting tablet, completely inactivated the carcinogenic acetaldehyde in the saliva during smoking. The L-cysteine tablet of 1.25 mg reduced the amount of acetaldehyde by about two thirds compared with the placebo.

### Example 5 (not according to the invention)

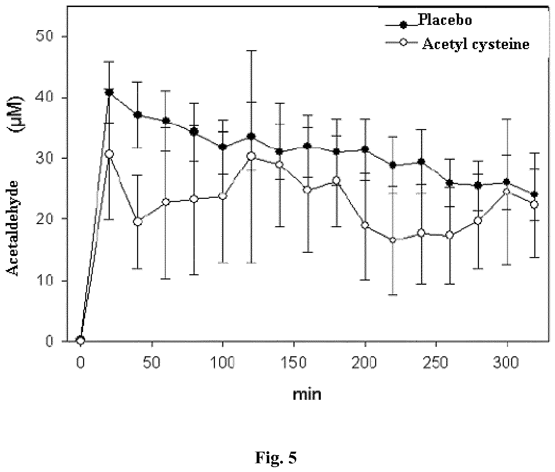

The testees enjoyed alcohol in amounts of 0.8 g per kilo of body weight. Thereafter, the testees attached, under their upper lip, a buccal tablet that contained 100 mg of N-acetyl cysteine and that slowly released the acetyl cysteine. The salivary acetaldehyde levels were measured at intervals of 20 minutes up to 320 minutes. The results are shown in Fig. 5. Throughout, the acetaldehyde contents of those using the acetyl cysteine tablets were lower than of those using the placebo.

## Claims

1. A sucking tablet comprising 1.25 to 5 mg of L-cysteine for use in reducing tobacco and/or alcohol dependence in an individual who is dependent on tobacco and/or alcohol, wherein
a) the individual sucks the tablet simultaneously with smoking a tobacco product and/or consuming alcohol,
b) the individual is allowed to smoke and/or drink alcohol, whereby the L-cysteine binds the acetaldehyde formed from the tobacco product and/or alcohol, thereby reducing the formation of harmane, and
c) stages a) and b) are repeated as many times that the reduction in the tobacco and/or alcohol dependence of the individual results in a cessation of smoking and/or consuming alcohol.

2. The sucking tablet for use according to claim 1, wherein the tablet releases L-cysteine during smoking of the tobacco product.

3. The sucking tablet for use according to claim 1, wherein the L-cysteine is released into the mouth for at least 30 minutes.

## Patentansprüche

1. Lutschtablette, umfassend 1,25 bis 5 mg L-Cystein zur Verwendung bei der Verringerung von Tabak- und/oder Alkoholabhängigkeit bei einer Person, die von Tabak und/oder Alkohol abhängig ist, wobei
a) die Person die Tablette gleichzeitig mit dem Rauchen eines Tabakprodukts und/oder Konsumieren von Alkohol lutscht,
b) der Person erlaubt wird, zu rauchen und/oder Alkohol zu trinken, wobei das L-Cystein den aus dem Tabakprodukt und/oder Alkohol gebildeten Acetaldehyd bindet, wodurch dabei die Bildung von Harman vermindert wird, und
c) Stufen a) und b) so viele Male wiederholt werden, dass die Verminderung der Tabak- und/oder Alkoholabhängigkeit der Person zu einer Entwöhnung des Rauchens und/oder Konsumierens von Alkohol führt.

2. Lutschtablette zur Verwendung nach Anspruch 1, wobei die Tablette während des Rauchens des Tabakprodukts L-Cystein freisetzt.

3. Lutschtablette zur Verwendung nach Anspruch 1, wobei das L-Cystein für mindestens 30 Minuten in den Mund freigesetzt wird.

## Revendications

1. Comprimé à sucer comprenant de 1,25 à 5 mg de L-cystéine pour une utilisation pour réduire la dépendance à l'égard du tabac et/ou de l'alcool chez un individu qui est dépendent au tabac et/ou à l'alcool, dans lequel
a) l'individu suce le comprimé simultanément avec une consommation d'un produit du tabac et/ou d'alcool,
b) l'individu est autorisé à fumer et/ou à boire de l'alcool, de sorte que la L-cystéine se lie à l'acétaldéhyde formé par le produit du tabac et/ou de l'alcool, empêchant ainsi la formation d'harmane, et
c) les étapes (a) ou (b), sont répétées autant de fois pour que la réduction de la dépendance au tabac et/ou à l'alcool chez un individu conduise à l'abandon du tabac et/ou de la consommation d'alcool.

2. Comprimé à sucer pour une utilisation selon la revendication 1, dans laquelle le comprimé libère la L-cystéine pendant la consommation du produit du tabac.

3. Comprimé à sucer pour une utilisation selon la revendication 1, dans laquelle le comprimé libère la L-cystéine dans la bouche pendant au moins 30 minutes.
